# EUROPEAN PATENT APPLICATION

(11) **EP 3 305 883 A1**
(43) Date of publication of application: **11.04.2018**
(21) Application number: 16799777.4
(22) Date of filing: 09.05.2016
(51) Int. Cl.: C12M 1/00

(54) **LIQUID CIRCULATION CONTAINER, CELL CONCENTRATION DEVICE AND CELL CONCENTRATION SYSTEM**

(30) Priority: 28.05.2015 JP 2015108924
(71) Applicant: Hitachi, Ltd., Tokyo 100-8280 (JP)
(72) Inventor: KIYAMA, Masaharu, Tokyo 100-8280 (JP); KATO, Midori, Tokyo 100-8280 (JP); HASHIBA, Shuhei, Tokyo 100-8280 (JP); IGARASHI, Yumiko, Tokyo 100-8280 (JP)
(74) Representative: Strehl Schübel-Hopf & Partner
(86) International application number: PCT/JP2016/063724
(87) International publication number: WO 2016/190069

(57) **Abstract**

The present invention is directed to provide cell concentration devices and cell concentration systems having a novel liquid flow-circulation chamber. It is a cell concentration device including a liquid flow-circulation chamber, the liquid flow-circulation chamber having a first tube and a second tube, the first tube and the second tube being connected with each other via a liquid delivery tube outside the liquid flow-circulation chamber, the liquid delivery tube including a liquid delivery pump and a cell concentration module, the liquid delivery pump being capable of feeding a liquid through the liquid delivery pump in both directions, the first tube and the second tube each having an open end in a liquid in the liquid flow-circulation chamber, the first tube having an opening in a gas in the liquid flow-circulation chamber, and the liquid being a suspension of cells.

## Description

### [Cross reference to related applications]

This application claims priority to Japanese Patent Application No. 2015-108924 filed on May 28, 2015, which is hereby incorporated by reference in its entirety.

### [Technical field]

The present invention relates to liquid flow-circulation chambers, cell concentration devices, and cell concentration systems.

### [Background art]

In regenerative medicine in which diseases are treated using autologous or allogenic cells, in order to use cells for transplantation therapy, it is necessary to increase the number of the cells or cause the cells to form a tissue in an appropriate structure by culturing the cells collected from a living body. Cell culture and preparation to be used for medical treatment should be performed in accordance with GMP (Good Manufacturing Practice) in a cell culture clean room called Cell Processing Facility (CPF). The problems here are that since the cell culture is performed manually by a technician, the preparation of cells for a single patient requires great effort and cost and that such manual implementation entails a risk of biological contamination and possible operational mistakes.

As means for solving these problems, devices with which cell culture and preparation processes can be automated in a closed system have been developed. In other words, by using a closed culture vessel which does not require any operation of opening and closing the lid of the culture vessel or by automating the cell culture process itself, it is possible to reduce operational mistakes and the risk of biological contamination.

In such systems, centrifugation and membrane concentration using a filtration membrane are used as methods of concentrating cells. In typical manual culture, the centrifugation is used for the purpose of, for example, increasing the concentration of a cell suspension or exchanging cell buffer. In centrifugation, the cell suspension is hermetically enclosed in a tube with a lid in a clean bench, and is separated into cell pellet and supernatant fractions by a centrifuge. The lid of the tube is removed in the clean bench and the supernatant is aspirated with an aspirator, taking care not to aspirate the cell pellet at the bottom of the tube. At this point in time, cell suspension is prepared by adding another buffer to a predetermined concentration. As can be seen from the above, due to the complexity of the series of steps, the centrifugation has problems of not only being difficult to be automated but also entailing expense for sterilizing instruments as well as an area for preparations because the preparation is done in an open system.

In recent years, the development of biopharmaceuticals is progressing which utilize secretions from cells as pharmaceutical agents like in the case of antibody medicines and vaccines, and separation of cells from a cell suspension is necessary in production of such pharmaceutical agents. Conventionally, centrifugation was used for this concentration, but production techniques for biopharmaceuticals using a single-use closed flow path have almost been established, among which membrane concentration by tangential flow filtration (hereinafter, abbreviated as TFF) has come to be widely used. The TFF membrane concentration takes a long time for processes and requires prearrangement steps for operation such as installation of flow paths and membrane modules in a closed system, but it has a simple control mechanism and can be automated easily. Examples of cell concentration devices using such TFF membranes are disclosed in JP-A-2015-12837 and WO2015/012174.

Automated manipulation of the cell suspension, however, results in the increase in damage to the cells as compared to manual manipulation of the cells. In view of this, Patent Document 1 discloses a method with which cells are less damaged, by passing a cell suspension through a cell suspension processor while controlling the linear velocity of the suspension to a specific range.

In culturing cells, a typical procedure involves adding a base component such as NaHCO₃ called a bicarbonate-based buffer to a liquid medium to be used and maintaining the mixture in a chamber of a CO₂ incubator where the concentration of CO₂ is as high as 5-10%, thereby controlling the pH value of the culture so as not to be varied significantly due to the contact between the medium and a gas phase. The pH value is, however, still likely to be varied if the medium frequently contacts the air (which is typically 0.04% CO₂, 20% O₂, and 78% N₂, and others). When a TFF membrane is used for concentration in a closed flow path, the pH value of the liquid medium is likely to be varied because it comes in contact with the air in a tube for delivering the cell suspension or in a container for holding it. (In this specification, the air refers to a gas having the same or substantially the same concentration as the aforementioned one, and the non-air gas refers to a gas having a composition different from that of the air.)

Further, in the closed flow path with which concentration with a TFF membrane is performed, air bubbles are easy to be formed due to drop fall or impact during the delivery of the cell suspension. Formation of bubbles in the cell suspension not only raises the possibility of changing the pH value of the medium due to the increase in frequency of contact between the medium and the air but also increases the chance of killing cells due to a sudden change in pressure between the medium and the air upon bubbling. In view of this, Patent Document 2 discloses a means for preventing the formation of bubbles by stopping the supply of a cell concentrate by detecting bubbles in the concentrate.

### [Summary of the invention]

### [Problems to be solved]

An object of the present invention is to provide a novel cell concentration devices and cell concentration systems having a liquid flow-circulation chamber.

### [Means for solving the problems]

An aspect of the present invention is a liquid flow-circulation chamber including a first tube and a second tube, the liquid flow-circulation chamber being hermetically closed, the first tube and the second tube being connected with each other via a liquid delivery tube outside the liquid flow-circulation chamber, the liquid delivery tube including a liquid delivery pump capable of feeding a liquid through the liquid delivery pump in both directions, the first tube and the second tube each having an open end in a liquid in the liquid flow-circulation chamber, and the first tube having an opening in a gas in the liquid flow-circulation chamber. It may include a third tube connected to a gas bag. An opening area of the opening may be larger than a cross-sectional area of the first tube. The liquid may contain cells.

Another aspect of the present invention is a cell concentration device including a liquid flow-circulation chamber, the liquid flow-circulation chamber having a first tube and a second tube, the first tube and the second tube being connected with each other via a liquid delivery tube outside the liquid flow-circulation chamber, the liquid delivery tube including a liquid delivery pump and a cell concentration module, the liquid delivery pump being capable of feeding a liquid through the liquid delivery pump in both directions, the first tube and the second tube each having an open end in a liquid in the liquid flow-circulation chamber, the first tube having an opening in a gas in the liquid flow-circulation chamber, and the liquid being a suspension of cells. It may include a third tube connected to a gas bag. The gas bag may contain a gas containing 5 to 10% carbonate gas, and the suspension of the cells may be a carbonate buffer. An opening area of the opening may be larger than a cross-sectional area of first tube. It may further include a discharge bag connected to the cell concentration module through an on-off valve or a check valve, the discharge bag being for holding a discharge from the cell concentration module.

A yet another aspect of the present invention is a cell concentration system including any cell concentration device described above in an attachable manner. It may further include a pressure sensor and/or a weight sensor. The pressure sensor may be at least one pressure sensor selected from a group consisting of a pressure sensor for detecting a pressure in the upstream of the cell concentration module, a pressure sensor for detecting a pressure in the downstream of the cell concentration module, and a pressure sensor for detecting a pressure of a filtrate of the cell concentration module. It may further include a sample container for supplying the cell suspension to the liquid flow-circulation chamber; and a collection container for collecting the concentrated cell suspension.

### [Brief description of the drawings]

Fig. 1 is (A) a configuration diagram of a cell concentration device, and (B) several examples of a shape of an opening in the distal end of an inlet tube, according to one embodiment of the present invention;
Fig. 2 is an example of a configuration diagram of a conventional cell concentration device;
Fig. 3 is a configuration diagram of a cell concentration device according to another embodiment to the present invention;
Fig. 4 is a configuration diagram of a cell concentration system according to one embodiment of the present invention;
Fig. 5 is a control flowchart of a cell concentration system according to one embodiment of the present invention;
Fig. 6 is a configuration diagram of a cell concentration system having a plurality of cell concentration devices according to one embodiment of the present invention;
Fig. 7 is a configuration diagram of a cell concentration system having a plurality of cell concentration modules according to one embodiment of the present invention; and
Fig. 8 is a table showing the results of cell concentration using a cell concentration device according to an example of the present invention or a conventional cell concentration device.

### [Embodiments for carrying out the invention]

Hereinafter, embodiments of the present invention are described in detail with reference to examples.

The objects, features, advantages, and ideas of the present invention will be apparent to those skilled in the art from the description of the present specification, and those skilled in the art can easily reproduce the present invention from the description of the present specification. Embodiments of the invention described below and specific examples show preferred embodiments of the present invention and are shown for the purpose of illustration or explanation, which do not limit the present invention thereto. It will be apparent to those skilled in the art that various changes and modifications can be made based on the description herein within the intention and scope of the present invention disclosed herein.

### <Liquid flow-circulation chamber>

The liquid flow-circulation chamber of the present invention is a liquid flow-circulation chamber including a first tube and a second tube, the liquid flow-circulation chamber being hermetically closed, the first tube and the second tube being connected with each other via a liquid delivery tube outside the liquid flow-circulation chamber, the liquid delivery tube including a liquid delivery pump capable of delivering a liquid through the liquid delivery pump in both directions, the first tube and the second tube each having an open end in a liquid in the liquid flow-circulation chamber, and the first tube having an opening in a gas in the liquid flow-circulation chamber. In the present specification, the "flow-circulation" used in terms of a flow-circulation chamber or liquid flow-circulation chamber means that the liquid in a chamber flows out of the chamber and some or all of the liquid that has flown out returns to the chamber.

The shape of the liquid flow-circulation chamber is not particularly limited, but a bottle-like shape is preferable in view of ease of handling. The material is not particularly limited and, for example, a material having liquid resistance, such as not being corroded by the liquid to be put in the chamber, is preferable, but a user can appropriately select the material depending on the way of use or the like.

The shape and size of the opening are not particularly limited, but examples of the shape include a circle, an ellipse, an oval, a square, and a rectangle. In the case of an elliptical shape, an oval shape, or a rectangular shape, it is preferable that the direction of the major axis of the first tube coincides with the direction of the major axis of the opening. In addition, in order to avoid production of aspiration force from the opening toward the outside of the first tube and avoid formation of bubbles at the time of inflow of the liquid, it is preferable that, the diameter of a circular opening, the smallest diameter passing through the center of an elliptical or oval opening, or the length of a short side of a square or rectangular opening, is larger than the inner diameter of the first tube, or the area of the opening that opens in the gas is larger than the cross-sectional area of the first tube. Depending on the viscosity of the liquid to be handled, a much larger opening is preferable. In addition, the number of the openings that open in the gas in the liquid flow-circulation chamber may be either one or more than one.

The first tube and the second tube are connected with each other via the liquid delivery tube outside the liquid flow-circulation chamber. Therefore, when the liquid delivery pump sends the liquid in the direction from the second tube to the first tube, the liquid that has flown out from the liquid flow-circulation chamber through the second tube flows into the liquid flow-circulation chamber through the first tube. On the other hand, when the liquid delivery pump sends the liquid in the direction from the first tube to the second tube, the air in the liquid flow-circulation chamber is aspirated first because the first tube has the opening that is opened in the gas, and accordingly the liquid in the liquid delivery tube is collected in the liquid flow-circulation chamber, and the liquid delivery tube is thus emptied. As long as the liquid delivery tube has this function, it may have any device. Examples of devices that may be included are, but not limited to, a valve, a filter, and a column. By providing such a device, it becomes possible to temporarily discharge the liquid in the liquid flow-circulation chamber to the outside for a desirable treatment to the liquid in an automatic manner and return it to the liquid flow-circulation chamber. It should be noted that not all the liquid that has flown out through the second tube needs to be returned through the first tube; instead, for example, the liquid delivery tube may branch midway, and some of the liquid may flow out through the branch of the liquid delivery tube and the remaining liquid may be returned to the liquid flow-circulation chamber.

As described above, by providing the first tube with an opening, it is possible to circulate the liquid in the liquid flow-circulation chamber in the direction from the second tube to the first tube and collect the liquid in the liquid delivery tube into the liquid flow-circulation chamber in the direction from the first tube to the second tube. By providing the first and second tubes with the open ends in the liquid in the liquid flow-circulation chamber, it is possible to collect the liquid gently through the first tube, reduce the contact between the liquid and the gas in the liquid flow-circulation chamber, and prevent bubbling of the liquid.

The liquid flow-circulation chamber may include a third tube connected to a gas bag, which allows gas to be supplied from the gas bag to keep the entire system closed when the pressure within the liquid flow-circulation chamber falls to negative. Furthermore, the space in the liquid flow-circulation chamber and the liquid delivery tube can be filled with the desired gas.

The liquid to circulate is not particularly limited. It may be a solution or a suspension, and may contain cells. The cells may be cultured cells or cells isolated from a living body, but it is preferable that the cells are alive. Since living cells are vulnerable to contact with gas such as bubbles, the configuration in which the first tube has an opening functions particularly effectively.

### <Cell concentration device>

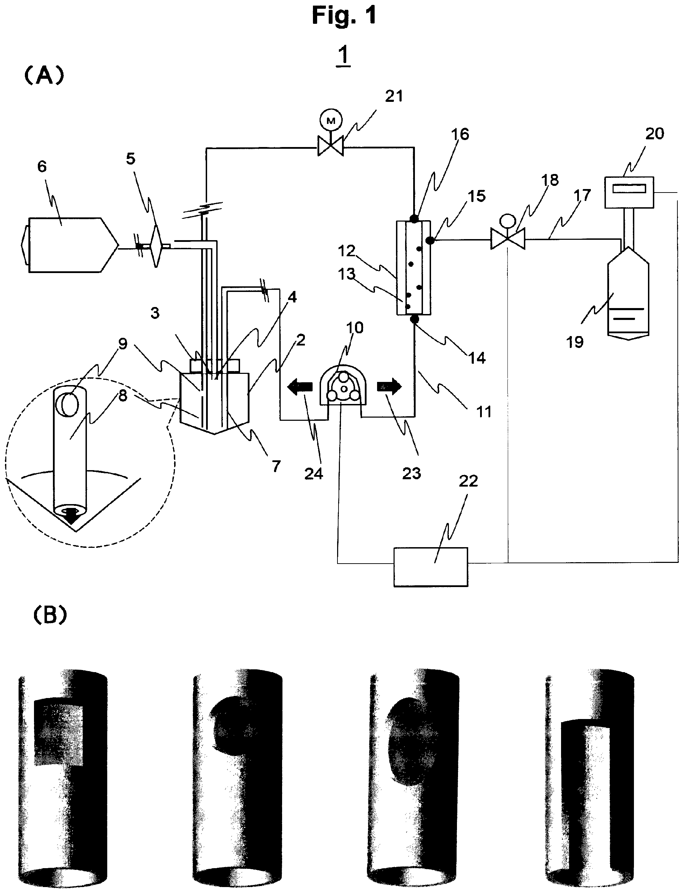

Fig. 1(A) is a diagram showing a configuration of a cell concentration device 1 in one embodiment.

This cell concentration device 1 is provided with a flow-circulation chamber 2, which is the aforementioned liquid flow-circulation chamber, for holding a cell suspension. The shape of the flow-circulation chamber 2 is not particularly limited, but if the chamber has a shape of a bottle, it is easy to put liquid in, is convenient because the volume of the liquid can be observed easily, and is user-friendly to handle at the time of collection so operational mistakes hardly occur. In addition, a chamber with a conical-shaped bottom is preferable because even a small volume of cell suspension can be aspirated and discharged.

This flow-circulation chamber 2 has a lid 3 with which the chamber can be made air-tight. The lid 3 may be provided with an atmospheric pressure regulating tube 4 for adjusting the atmospheric pressure. The atmospheric pressure regulating tube 4 is connected to a gas bag 6 through a filter 5 having a mesh size of 0.22 µm. In the gas bag 6, a necessary volume of non-air gas for preventing qualitative change of the cell suspension is contained beforehand. The non-air gas contains preferably 5-10% CO₂. The shape of the gas bag 6 is not particularly limited, but one having a sack-like shape is preferable, which makes it easy to, in filling the non-air gas, hold it at a desired concentration in the volume of the bag by squeezing the bag to discharge the gas from inside the bag beforehand and then filling the bag with the desired non-air gas. For a material of the gas bag, a resin material is suitable, and because of the necessity of holding the non-air gas in the gas bag, it is preferable that the gas bag has a low permeability to gas, and ethyl vinyl alcohol, polyester, polyvinyl chloride or the like is used.

The lid 3 of the flow-circulation chamber 2 is further provided with an aspiration tube 7 and an inlet tube 8. The aspiration tube 7 has an open end inside the flow-circulation chamber 2, and its length in the flow-circulation chamber 2 is adjusted so as to open in the cell suspension held in the flow-circulation chamber 2. Similarly, the inlet tube 8 also has an open end inside the flow-circulation chamber 2, and its length in the flow-circulation chamber 2 is adjusted so as to open in the cell suspension held in the flow-circulation chamber 2. The inlet tube 8 is further provided with an opening 9 which opens to the gas in the flow-circulation chamber 2.

The shape, size, and number of the opening 9 are not particularly limited, and what is described for the aforementioned opening in the liquid flow-circulation chamber can be applied to the opening 9. Some examples of the shape of the opening 9 are shown in Fig. 1(B).

By configuring the inlet tube 8 in this way, when the cell suspension flows into the flow-circulation chamber 2 through the inlet tube 8, the cell suspension flows from the open end directly into the cell suspension held in the flow-circulation chamber 2. When the cell suspension is aspirated from the flow-circulation chamber 2 through the inlet tube 8, the gas in the flow-circulation chamber 2 flows out of the flow-circulation chamber 2 through the opening 9.

A pump 10 is provided in a liquid delivery tube 11 connected to the aspiration tube 7 to adjust the flow of the cell suspension in the liquid delivery tube 11. The pump 10 is preferably a roller pump but it is not particularly limited, and a pump of a different type may be used such as a diaphragm pump, a gear pump, a peristaltic pump, and a tube pump. In the cell concentration device, since it is necessary to ensure the sterility of the tube, it is preferable that the inside of the liquid delivery tube can be sterilized before use. A roller pump in which the tube can be replaced in every use is particularly preferable.

The liquid delivery tube 11 is provided with a cell concentration module 12 for concentrating the cells in the cell suspension. The cell concentration module 12 has a separation membrane 13 which is a hollow fiber membrane formed into a tube, and has an outer cylindrical member on the outside thereof. The cell suspension before concentration is supplied to the separation membrane 13 through the inlet port 14 connected to the liquid delivery tube 11, and separated into a filtration port 15 through which filtered components pass and an outlet port 16 for the liquid that has not been filtered and left.

The filtration port 15 is connected to an on-off valve 18 for controlling a filtration tube 17, and the filtration tube 17 is connected to a discharge bag 19. The filtrate which is a liquid discharged from the cell concentration module 12 is eventually held in the discharge bag 19. The discharge bag 19 may be provided with a weight sensor 20, which measures the weight of the discharged filtrate and the amount of the filtrate is calculated from the density of the filtrate, thereby a flow rate per unit time is measured. As will be described later, the on-off valve 18 may be a check valve.

On the other hand, the liquid delivery tube 11 connected to the outlet port 16 of the cell concentration module 12 is connected to a pressure regulating valve 21, and is connected to the inlet tube 8 of the flow-circulation chamber 2 therefrom.

The tubes used for the liquid delivery tube 11 and the filtration tube 17 in the cell concentration device 1 are suitably made of resin materials having flexibility and preferably having low permeability to gas because of the necessity of passing the non-air gas there through. Ethyl vinyl alcohol (EVOH), polyester, polyvinyl chloride and the like are suitably used. Furthermore, in the on-off valve, it is necessary to use a rubber material having high elasticity for opening and closing a connection tube with a solenoid valve. Rubber materials having low permeability to gas or chemically synthesized rubbers can be used.

Thus, a flow-circulation path is formed in which the cell suspension held in the flow-circulation chamber 2 is supplied to the cell concentration module 12 through the aspiration tube 7 with the pump 10 serving as a driving source, concentrated in the cell concentration module 12, discharged from the outlet port 16 of the cell concentration module 12 and returned to the flow-circulation chamber 2 through the inlet tube 8. The pressure regulating valve 21 adjusts the pressure in this flow-circulation path. Then, a controller 22 controls the pump 10, the on-off valve 18, and the pressure regulating valve 21 with good timing.

Since this cell concentration device is a closed system, it can be used without any risk of bacterial contamination. For example, before use, the cell concentration device in which the flow-circulation chamber 2, the filter 5, the gas bag 6, the tubing of the pump 10, the liquid delivery tube 11, the cell concentration module 12, the filtration tube 17, the discharge bag 19, and the tubing of the pressure control valve 21 are connected via connection tubes is taken from, for example, a sterile bag in a sterile space such as a clean bench and a sample to be concentrated is put in the flow-circulation chamber 2. Then the lid is closed and the closure of the flow path is checked, and then the cell concentration device is mounted onto a device equipped with a driving source such as a pump. Thus, it is possible to perform the concentration step while maintaining the closed environment by

### <Concentration of cells using cell concentration device>

A method of concentrating cells using such a cell concentration device is described below.

First, the cell suspension to be concentrated is put in the flow-circulation chamber 2. At this time, the volume of the cell suspension is determined so that its surface is at a level higher than the open ends of the aspiration tube 7 and the inlet tube 8 to allow the aspiration tube 7 and the inlet tube 8 to be directly put in the cell suspension. Furthermore, a part or the whole of the opening 9 of the inlet tube 8 is located at a position higher than the liquid surface of the cell suspension, so that a part or the whole of the opening 9 is exposed to the gas over the cell suspension. It is, however, preferable that the entire opening 9 is located at a level higher than the liquid surface of the cell suspension.

When the flow-circulation chamber 2 is hermetically closed by closing the lid 3 of the flow-circulation chamber 2 and the on-off valve 18 for controlling the filtration tube 17 is opened to drive the pump 10 in a liquid delivery direction depicted by an arrow 23, the cell suspension held in the flow-circulation chamber 2 is delivered to the outlet port 14 of the cell concentration module 12. By holding the cell suspension at a liquid delivery pressure suitable for concentrating cells, a liquid medium containing factors secreted from the cells or serum components is separated from the cell suspension in the cell concentration module 12. These separated substances are discharged through the filtration port 15 of the cell concentration module 12 while the cell suspension (cell concentrate) concentrated in the cell concentration module 12 returns to the flow-circulation chamber 2 through the inlet tube 8. At this time, since the open end of the inlet tube 8 is in the cell suspension in the flow-circulation chamber 2, the cell concentrate gently flows, without forming bubbles, into the cell suspension held in the chamber. In this way, by using the flow-circulation chamber 2 having the configuration according to this embodiment, it is possible to prevent formation of bubbles.

When the cell suspension in the flow-circulation chamber 2 reaches a desired liquid volume, the delivery of the liquid for concentration is stopped. At this point, since a large volume of the cell suspension is held in the flow-circulation flow path including the cell concentration module 12, the cell suspension within the flow-circulation chamber 2 can be collected by reversing the direction of liquid delivery 24 of the pump 10 relative to the direction during the concentration. By closing the on-off valve 18 for controlling the filtration tube 17 and opening the pressure regulating valve 21 beforehand in order to prevent the filtered liquid from flowing backward, the cell suspension in the flow-circulation flow path can be returned to the flow-circulation chamber through the aspiration tube 7 with the gas supplied from the opening 9. This makes it possible to increase a cell recovery rate. Using a check valve for the on-off value 18 eliminates the necessity of controlling the opening and closing of the on-off valve 18 as described above.

Furthermore, when the volume of the liquid in the flow-circulation chamber 2 decreases, a non-air gas for preventing qualitative change (e.g., change in pH) of the liquid held at the atmospheric pressure is replenished from the gas bag 6 through the atmospheric pressure adjustment tube 4, and the flow-circulation chamber 2 is filled. For example, when the cell suspension is a cell culture medium or a cell buffer (for example, MEM or DMEM, or Hanks buffer or HEPES buffer) which is a bicarbonate-based buffer, the change in pH of the cell suspension can be limited by using 5-10%CO₂ as the non-air gas depending on the strength of the carbonate buffer.

### <Reference Example 1>

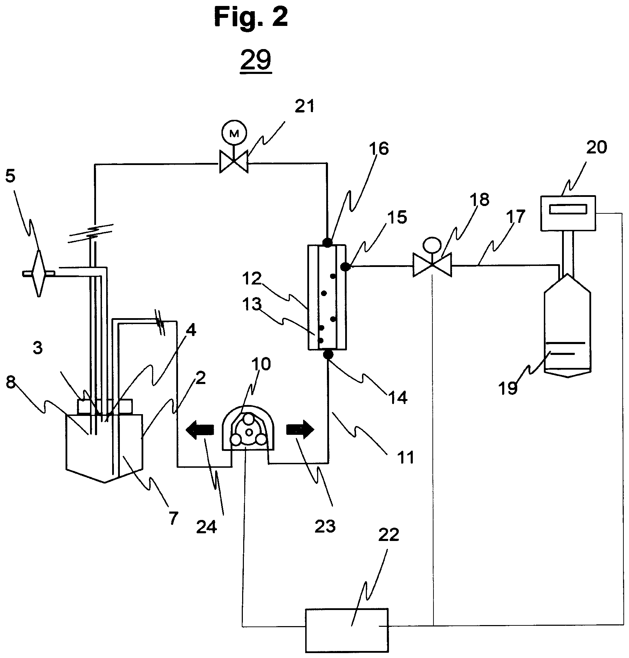

Fig. 2 shows an example of a conventional cell concentration device 29 which has a flow-circulation chamber 2 with a conventional form. Since the inlet tube 8 in the flow-circulation chamber 2 does not have the opening 9 of Fig. 1, the open end of the inlet tube 8 is open in the gas and thus the liquid falls from the open end of the inlet tube 8 into the cell suspension. At that time, splashes occur on the liquid due to the drop impact, and air bubbles are formed due to the air at the liquid surface entrained in the cell suspension at the bottom. When the opening 9 of the inlet tube 8 in the flow-circulation chamber 2 is located near the cell suspension or in the cell suspension, the cell concentrate in the flow-circulation chamber 2 can be aspirated and circulated again upon the delivery of the liquid by the pump 10 in the direction of liquid delivery 24.

### <Reference Example 2>

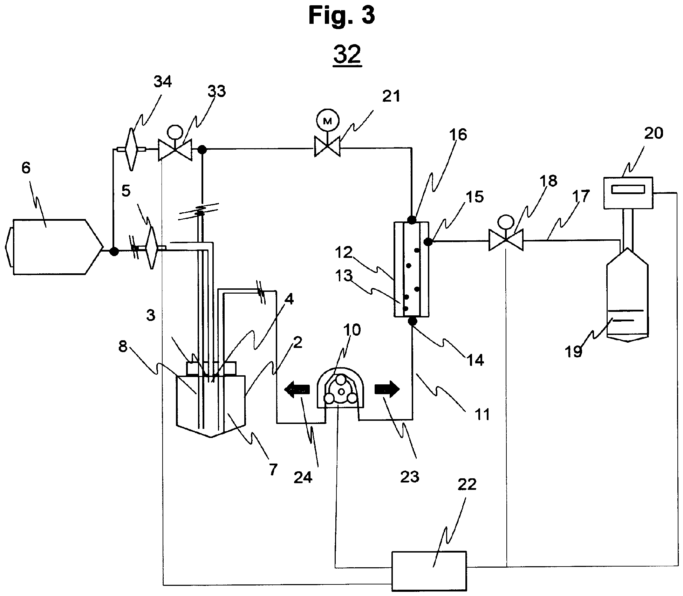

Fig. 3 shows a cell concentration device 32 shown as another embodiment. The inlet tube 8 does not have an opening. The open end thereof is opened in the cell suspension held in the flow-circulation chamber 2. The liquid delivery tube 11 is branched and an on-off valve 33 and a filter 34 are provided. Furthermore, a connection tube is connected to a gas bag 6, and the non-air gas in the gas bag 6 is used for regulating air pressure in the flow-circulation chamber 2 and the intake of gas phase when the liquid is delivered by the pump 10 in the direction of liquid delivery 24. This method makes it possible to prevent bubble formation in the inlet tube 8 and limit the inflow of the gas phase into the flow-circulation chamber 2. As compared with the first embodiment, however, the configuration of the flow path is complicated and the addition of the on-off valves 33 results in the increase of requirements to be controlled.

### <Cell concentration system>

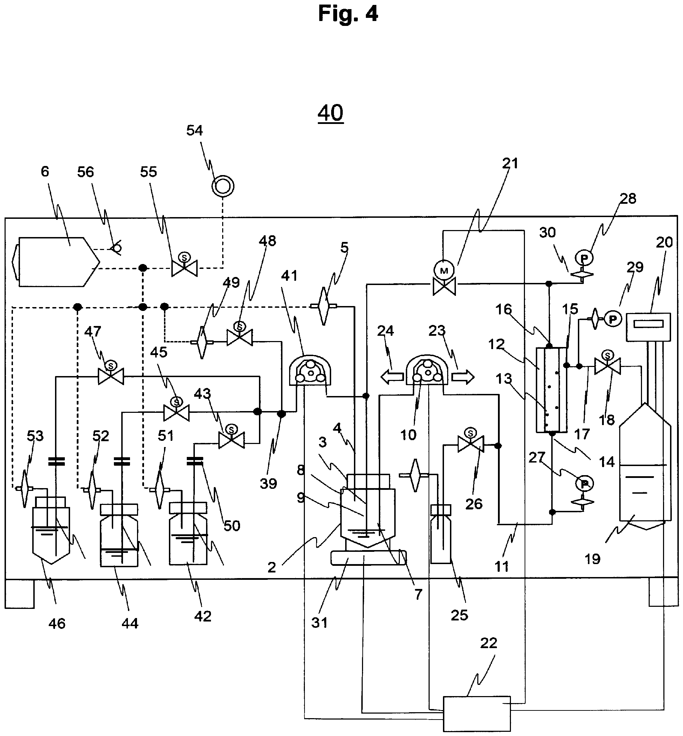

A cell concentration system using the cell concentration device of the present invention is described. Fig. 4 shows a configuration of a cell concentration system 40 in which the cell concentration device 1 is incorporated to perform a cell concentration step in a fully automated manner. This device performs, in a fully automated manner, a step of filling a non-air gas with which qualitative change in liquid is prevented, as a first step; a step of delivering a washing buffer to condition and wash the cell concentration module before cell concentration, as a second step; a step of concentrating the cell suspension in the cell concentration module to produce a cell concentrate, as a third step; a step of delivering other buffer to dilute the cell concentrate with the other buffer, as a fourth step; and a step of collecting the cell concentrate in a cell concentrate collecting vessel, as a fifth step.

As shown in Fig. 4, the present system is composed of an attachable cell concentration device and a mechanism for holding and controlling it. Basically, the device in Fig. 1 is used for the cell concentration device. In this system, the connection tube between the pump 10 and the cell concentration module 12 is branched and connected to a collection bottle 25 which is the cell concentrate collection vessel, and an on-off valve 26 for controlling this connection tube is provided.

The cell concentration system 40 may be provided with a pressure sensor for detecting the pressure in a cell flow path. For example, a pressure sensor may be provided to detect the pressure in the flow path for the cell suspension (which is herein also referred to as a cell flow path) and placed in the connection tube between the cell concentration module 12 and the pump 10. Specifically, for example, three pressure sensors, i.e., a pressure sensor Pf ("f" means "feed") 27 for detecting the pressure in the upstream of the cell concentration module 12, a pressure sensor Pr ("r" means "retentate") 28 for detecting the pressure in the downstream of the cell concentration module, and a pressure sensor Pp ("p" means "permeate") 29 for detecting the pressure of the filtrate of the cell concentration module, may be provided and all of them may be connected through a hydrophobic filter 30. The controller 22 that adjusts the pressure in the circulating flow path receives information from the pressure sensors and controls the flow rate of the pump 10 and the pressure regulating valve 21, based on the information. Thus, this system 40 can control trans-membrane pressures within the cell flow path to adjust shear rates during cell concentration and concentrating cells in a highly reproducible manner as well as control the volume of liquid to be delivered to ensure reproducibility and conform to the GMP standard. In addition, the pressure sensor is useful because it is possible to interrupt the concentration process safely by detecting an abnormal operation of the device. Conceivable examples of the abnormal operation of the device include detachment of a junction, abrupt drop of pressure due to a breakage of the flow path, and clogging and obstruction due to the concentrated substance in a connection tube. These events are detected as abnormal operations and the cell concentration step can be promptly stopped and shifted to an appropriate processing including whether the processing can be continued or not. In the cell concentration step, excessive concentration results in a significant increase in damage to the cells. In order to prevent this phenomenon, it is essential to monitor the pressure in the flow path. For example, when the liquid in the flow-circulation chamber 2 decreases and the cell concentrate with entrained gases flows out, the gas cannot pass through the filtration port in the cell concentration module and therefore accumulates on the cell concentration module. As a result, the pressure sensors Pf and Pp will soon show high values.

Similarly, the cell concentration system 40 may be provided with a weight sensor for monitoring change in flow velocity in the flow path in order to prevent excessive concentration as in the above case. The weight sensor detects the filtration speed and, for example, when the liquid concentrate in the flow-circulation chamber 2 decreases and the cell concentrate with entrained gases flows out, an extreme decrease in change of weight of the flow-circulation chamber 2 is detected. It is also possible to detect abnormality based on information on the amount of change of weight of the discharge bag 19. For example, the weight sensor 31 may measure the weight of the flow-circulation chamber 2 and calculate the holding amount from the density of the liquid held in the flow-circulation chamber 2 to measure the flow rate per unit time. In this manner, the system may be provided with the function of performing the concentration process more safely by detecting abnormality of the flow path by the monitor weight sensor.

The connection tube coupled to the inlet tube 8 of the flow-circulation chamber 2 is branched and connected to a discharge port of the pump 41. A aspiration port of the pump 41 is connected to bottles via an on-off valve 43 for opening and closing the connection tube of a wash bottle 42 for holding washing buffer, an on-off valve 45 for opening and closing the connection tube of a dilution buffer bottle 44 for holding dilution buffer, and an on-off valve 47 for opening and closing the connecting tube of a sample bottle 46 which is a sample container for holding the cell suspension. The connection tube connected to the aspiration port of the pump 41 is branched at a branch point 39, and connected to the gas bag 6 via an on-off valve 48 for opening and closing the connection tube and a filter 49. It should be noted that the wash bottle 42, the dilution buffer bottle 44, and the sample bottle 46 connected to the on-off valves 43, 45 and 47, respectively, are connected using a sterile connection part 50 that allows them to be connected in sterile conditions between tubes even in a general experimental environment.

The gas bag 6 is connected, through the filter 51 connected by a connection tube through the filter 5 to the atmospheric pressure regulating tube of the wash bottle 2, the filter 52 connected to the atmospheric pressure regulating tube of the dilution buffer bottle 44, and the filter 53 connected to the atmospheric pressure regulating tube of the sample bottle 46, to their own bottle Additionally, the gas bag 6 is connected to a non-air gas supply source 54 via the connection tube having an on-off valve 55, and has a check valve 56. In Fig. 4, the tubes connected to the gas bag 6 are depicted by broken lines as tubes through which only gases flow.

In the system 40, the cell concentration device may be integrally held by a mounting plate (not shown), sterilized by, for example, gamma-irradiation after assembling the cell concentration device, and installed in a main system before use. On the other hand, the main system is composed of motor units as driving sources in the pumps 10 and 41, electromagnetic valve units in the on-off valves 18, 26, 43, 45, 47, 48, and 55, pressure sensitive units in the pressure sensors 27, 28, and 29, and weight sensing units in the weight sensors 20 and 31. These mechanical elements and the cell concentration device are attachable, which allows single use or sterile cell concentration.

### <Method of concentrating cells using cell concentration system>

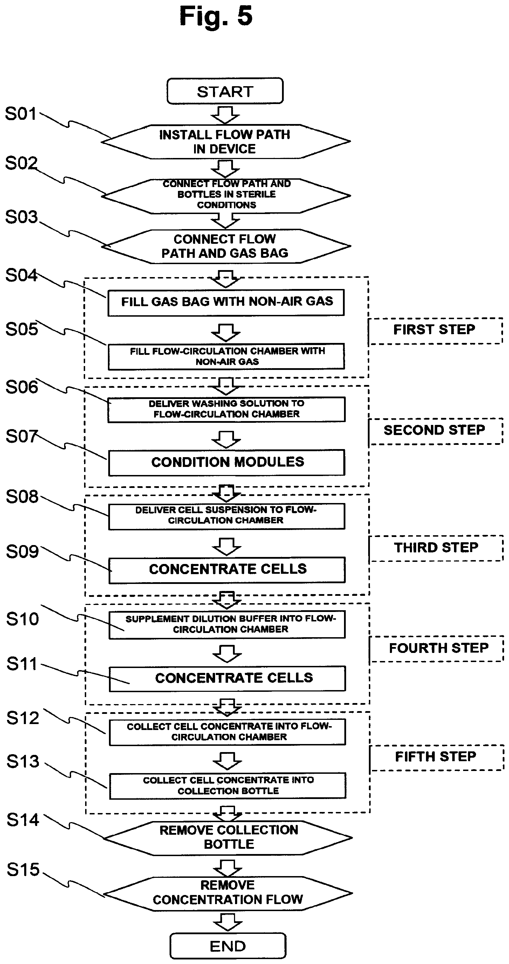

Fig. 5 shows a working process for concentrating a cell suspension by the cell concentration system 40. In this system, as a prearrangement step for operation, the cell concentration device is installed in the cell concentration system 40 (S01); the washing buffer bottle 42, the dilution buffer bottle 43, and the sample bottle 46 are connected to the sterile connection part 50 in the cell concentration device in sterile conditions (S02); and the filters 5, 51, 52, 53, and 49 are mounted onto the connecting tubes (S03).

Next, in the first step where a non-air gas (e.g., 5-10%CO₂) for preventing qualitative change of the liquid in the concentration step is filled, the on-off valve 55 is left open for a predetermined time to fill the gas bag 6 with the non-air gas (S04). Next, at a predetermined time after the on-off valve 26 is opened, the pump 10 is driven in the direction of liquid delivery 23 to thereby fill the flow-circulation chamber 2 with the non-air gas from the gas bag 6 (S05). Next, in the second step where the cell concentration module is conditioned and washed, a washing solution is delivered to the flow-circulation chamber 2 by the pump 41 (S06). Next, the washing solution in the flow-circulation chamber 2 is delivered to the cell concentration module 12 and the washing buffer is delivered to the discharge bag as the filtrate (S07). In the third step where cell concentrate is produced, the cell suspension in the sample bottle 46 is delivered to the flow-circulation chamber 2 (S08). Next, the cell suspension in the flow-circulation chamber 2 is delivered to the cell concentration module 12, and the components of the culture medium or serum are delivered to the discharge bag as the filtrate (S09). In the fourth step where the cell concentrate is re-suspended in another buffer, the dilution buffer in the dilution buffer bottle 44 is delivered to the flow-circulation chamber 2 (S10). Next, the dilution buffer in the flow-circulation chamber 2 is delivered to the cell concentration module 12, and the diluted components of the culture medium or serum are delivered to the discharge bag (S11). In the fifth step where the cell concentrate is collected, the cell concentrate in the flow path is delivered to the flow-circulation chamber 2 (S12). Next, the cell concentrate in the flow-circulation chamber 2 is delivered to the collection bottle 25 (S13).

As a step after the concentration operation, the collection bottle 25 holding the cell concentrate is removed from the cell concentration device (S14), and then the cell concentration device is removed from the cell concentration system 40 (S15) to finish the process.

In concentrating the cells at S09, when the on-off valve 18 that controls the filtration tube 17 is opened and the pump 10 is driven in the direction of liquid delivery depicted by the arrow 23, the cell suspension held in the flow-circulation chamber 2 is delivered to the outlet port 14 of the cell concentration module 12. By holding the cell suspension at a liquid delivery pressure suitable for concentrating cells, liquid medium and serum components for maintaining cells are separated from the cell suspension. These separated components are discharged through the filtration port 15 of the cell concentration module 12 while the cell concentrate is returned to the flow-circulation chamber 2 through the inlet tube 8. At this time, since the open end of the inlet tube 8 is in the cell suspension in the flow-circulation chamber 2, the cell concentrate gently flows from the open end of the inlet tube 8. As a result, it is possible to prevent bubbling in the flow circulation chamber 2 with the minimum damage to the cells.

The cell concentration system 40 may perform an automatic concentration step as follows. That is, the trans-membrane pressure (TMP) which is an average of pressure differences across the membrane is represented as TMP = (Pf + Pr)/2 - Pp, where Pf is a feed pressure, Pr is a circulatory pressure, and Pp is a permeate pressure. In typical membrane concentration, the concentration starts with a fixed TMP value suitable for each cell to be concentrated. The filtration rate decreases as the concentration proceeds to some degree and the cells in the sample are caught and layered on the surface of the filtration membrane. In that case, the delivery of the liquid is controlled with optimum TMP values for filtration by cooperatively controlling the pressure regulating valve 21 or controlling the flow rate of the pump. Alternatively, it is also possible to avoid formation of a layer by closing and opening the on-off valve 18 on the filtration side, which is called flushing. The weight sensor 20 for detecting the volume of the filtrate detects the change in weight per unit time as a filtration rate and it may be recorded and held as filtration information to determine whether or not the flushing is to be performed.

The cell concentration system 40 may quantify the liquid delivery as follows. Let a target feed volume be V. In an example of delivering the washing buffer, when the on-off valve 43 is opened and the pump 41 is driven, the gas in the connection tubes is delivered and the washing buffer in the wash bottle 42 which follows the gas passes through the connection tubes and thus the liquid delivery starts. The washing buffer passes through the pump 41 and reaches the flow-circulation chamber 2. The weight sensor 31 capable of measuring the weight of the flow-circulation chamber 2 measures the weight of the bottle depending on the volume of the liquid that has been delivered. The volume of the connection tubes from the branch point 39 to the pump 41 and to the inlet tube 8 in the flow-circulation chamber 2 is previously known and let the volume be A. The operation of the pump is interrupted when the weight sensor 31 detects a value of (V - A). Next, when the on-off valve 48 is opened, the washing buffer in the connection tube from the branch point 39 to the wash bottle 42 returns to the washing buffer bottle 42 by the potential energy due to the difference in height. The tube is closed by the internal structure of the pump 41 and no liquid therein moves. Next, when the pump 41 is activated for a predetermined time (discharge time), the non-air gas is introduced successively through the filter 49 and the washing buffer (volume A) temporarily remained in the connection tube is moved to the flow-circulation chamber 2, and the delivery of the liquid in a volume corresponding to the target liquid volume V is completed. The weight sensor 31 can be configured so that it can detect and record the weight of the liquid at the time of completion and record all the cell concentration conditions such as the feed rate and the feeding time.

### <Cell concentration system having a plurality of cell concentration devices>

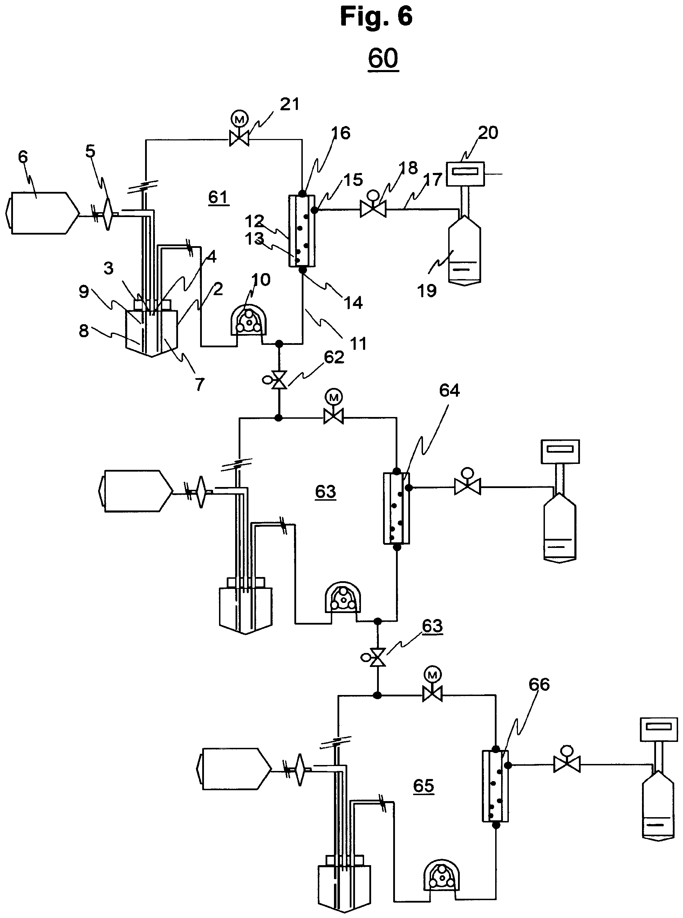

Fig. 6 is an embodiment of a cell concentration system 60 having a plurality of cell concentration devices aimed at further reducing the intake of the components unnecessary for the cell concentrate. In this embodiment, a first cell concentration device 61 is connected to a second cell concentration device 63 through the connection tube, with an on-off valve 62, branched from the connection tube 11 between the pump 10 and the cell concentration module. Likewise, the second cell concentration device 63 is connected to a third cell concentration device 65 through an on-off valve 64. As each cell concentration device, the cell concentration device that has been described in detail can be used.

This cell concentration system 60 can be used as follows.

In the first cell concentration device 61, the cell suspension is added to the flow-circulation chamber 2 and the cell suspension is concentrated to obtain a first cell concentrate. Next, when the on-off valve 62 is opened, the cell concentrate in the flow-circulation chamber 2 of the first cell concentration device 61 is delivered to the flow-circulation chamber 2 of the second cell concentration device 63 by the pump 10. If a predetermined volume of dilution buffer is held beforehand in the flow-circulation chamber 2 of the second cell concentration device 63, it is possible to reduce an amount of intake of unnecessary components. The reason is that, in the separation using a cell concentration module, target substances to be separated are often similar in molecular weight and/or particle diameter to components that should be removed, and thus the components that should be removed are left in the cell concentration module and cause clogging and reduction of the filtration rate. Accordingly, in order to reduce the amount of such intake, the concentrate is re-suspended in another dilution buffer and concentration is repeated, which leads an extension of the concentration time. According to Fig. 6, by concentrating, in a cell concentration device having an unused cell concentration module, the concentrated cells that have been concentrated in the cell concentration device, the unnecessary components that should be removed will be left in the cell concentration module of the used cell concentration device or left inside the connection tubes and/or the flow-circulation chamber. Therefore, the amount of intake can be reduced.

It is also possible to use the cell concentration module 12 and valves 64 and 66 in each cell concentration device for different cell concentration modules having different functions of separation.

### <Cell concentration system having a plurality of cell concentration modules>

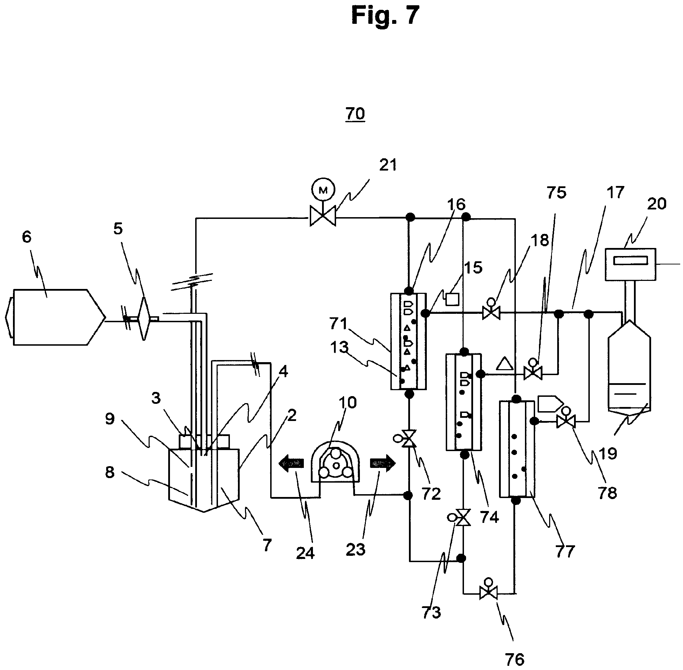

This embodiment shows a cell concentration system 70 having a plurality of cell concentration modules aimed at further reducing the intake of the components unnecessary for the cell concentrate. For a cell concentration module 71, a cell concentration module similar to the one in the aforementioned cell concentration device 1 is used. The basic configuration of the cell concentration device follows the one shown in Fig. 1, but the cell concentration device in this embodiment has a plurality of cell concentration modules. Now, referring to Fig. 7, an embodiment with three cell concentration modules is described. The number of the cell concentration modules can, however, be two or more, and four or more cell concentration modules may be provided in a manner similar to Fig. 7.

Specifically, an on-off valve 72 is provided in the connecting tube 11 between the pump 10 and the cell concentration module, and a second cell concentration module 74 is connected, through an on-off valve 73, to a connection tube that is branched at a point immediately upstream of the on-off valve 72. Similarly, a third cell concentration module 77 is connected, through an on-off valve 76, to a connection tube that is branched at a point immediately upstream of the on-off valve 73. By repeating such connection, it is possible to connect four or more cell concentration modules. In addition, each cell concentration module is provided with a filtration port, and also provided with the on-off valve 18 for controlling the filtration tube in a first cell concentration module, an on-off valve 75 for controlling the filtration tube in a second cell concentration module, and an on-off valve 78 for controlling the filtration tube in a third cell concentration module. These filtration tubes are connected to a discharge bag.

The cell concentration system 70 of this embodiment is used as follows. First, the cell suspension is held in the flow-circulation chamber 2 and is concentrated using only the first cell concentration module 71. The concentration of the cell suspension at this point in time is such that the volume of the suspension does not reach a final target volume (e.g., reduced to about 1/3 of the expected reduced volume). Next, by closing the on-off valve 72 and opening the on-off valves 73 and 75, the cell suspension is further concentrated using only the second cell concentration module 74. The concentration of the cell suspension at this point in time is again such that the volume does not reach the final target volume (e.g., reduced to about 2/3 of the expected reduced volume). Then, by closing the on-off valve 73 and opening the on-off valves 76 and 78, the cell suspension is further concentrated using only the third cell concentration module 77. When the cell suspension is concentrated to the final target volume, the cell concentration step is finished.

With such a cell concentration system, by further concentrating, using an unused cell concentration module, the cell suspension that has been concentrated to some extent, unnecessary components that should be removed are left in the used cell concentration module(s) and it is possible to remove the unnecessary components in a more efficient manner.

It is also possible to use the cell concentration modules 71, 74, and 77 in this cell concentration system for different cell concentration modules having different functions of separation.

### [Examples]

Hereinafter, an example of a method of concentrating cells which is applied to cancer cells using the cell concentration system in Fig. 4 is described.

### <Configuration of cell concentration device>

As the cell concentration modules 12, a MidiKros hollow fiber membrane module, Part Number D02-S500-05-P, membrane area 190 cm², molecular weight 500 kDa) which is a TFF cell concentration module manufactured by Spectrum, was used.

For each of solenoid valves in the on-off valves 18, 26, 43, 45, 47, 48, and 55, pinch valves (fluid pressure 0.2 MPa, Takasago Electric Inc., model PM-1015W) were used. For connection tubes corresponding to the solenoid valves, elastomeric tubes (inner diameter 1/16 in., outer diameter 1/8 in., Saint-Gobain, model number AY242002) were used. For each pump, a tube pump (discharge/aspiration pressure +/- 0.1 MPa, Welco Co., Ltd., model number WP1000-P3.2DM4-C) and an elastomeric tube (inner diameter 1/8 in., outside diameter 1/4 in., Saint-Gobain, model number AY242007) as a peristaltic tube were used in combination. Since this product has a roller unit that can be attached to and removed from a motor unit of the body, it is possible to sterilize the product with the elastomeric rubber tube (20.5 cm in length) wound on the roller unit. The flow rate of the pump was, at DC 12 V input, 1 mL/sec. according to the measurement results. For tubes other than the closure area of the solenoid valve and peristaltic area of the pump unit, Tygon ND-100 (inner diameter 1/16 in., outer diameter 1/8 in., Saint-Gobain, model #ADF00002) made of vinyl chloride was used. For branches and junctions of the tubes, SMC coupling (CPC Corp.) series was used. Specifically, Y Fitting was used for joining two branches (joint diameter 1/16 in., model #HY291) and Straight Fitting was used for straight connection (joint diameter 1/16 in., model #HS291).

For the flow-circulation chamber 2, a 500-mL centrifuge tube (Corning, Inc., model No. 11750) equipped with accessories was used with some modification. For the washing buffer bottle 42 and the dilution buffer bottle 44, NUNC graduated square, clear bottles (500 mL, model number 2015-0500JP) available from Thermo Fisher Scientific Inc. were used. For the discharge bag 19, Flexboy bag (double layered with EVA and EVOH, volume of 3L, Sartorius, model #FFB102812) was used.

For the collection bottle 25, a 50-mL centrifuge tube (Corning, Inc., model No. 11705) equipped with accessories was used. This product is made up of a container unit, a lid unit, conduits provided in the lid unit for gas pressure adjustment, and a filter of a mesh size of 0.22 µm, which are all pre-sterilized.

For the gas bag 6, Flexboy bag (double layered with EVA and EVOH, volume of 1L, Sartorius, model #FFB103547) was used. For the check valve 56 connected to a single lure port, a check vale (ARAM Corporation, model #PRC15, cracking pressure 0.2 to 1.5 kPa) was used.

For each of the filters 5, 49, 51, 52, and 53 and the hydrophobic filter 30, a vent filter (ZenPURE Corporation, model number D25CUE020LFLM) was used. For each of the sterile connections, ASPEEPQUICK S model number AQS17002 available from Colder Products Company was used in pairs.

The aforementioned components were assembled aseptically in a safety cabinet to create a cell concentration device. Then, after the cell concentration device was placed in a sterile bag and hermetically enclosed, it was subjected to gamma sterilization with 15 KGry.

### <Configuration of cell concentration system>

To each pressure sensitive part of the pressure sensors 27, 28, and 29, a pressure transducer (Spectrum, Inc., Model ACPM-799-01N) and a load cell amplifier (M-SYSTEM Co., Ltd., model number M2LCS-155-R/K/N) were connected to measure pressures. For the weight sensor 20, a load cell of a platform scale type (A & D Co., Ltd., model number LC4101-K1.5) and an amplifier indicator (A & D Co., Ltd., model number AD-4513B) were used. For the weight sensor 31, an upper tray electronic balance (A & D Company, model number EJ-610) was used. For the controller, a control controller (Keyence Corp., KV-5000 series) was used for automatic control.

### <Preparation of cells>

2.0 × 10⁷ cancer cells (HepG2 cell line) were dispersed on 500 ml (cell concentration of 4.0 × 10⁴ cells/ml) of growth medium (DMEM supplemented with 10% fetal calf serum and 1% each of penicillin-streptomycin) as a cell suspension.

### <Automatic concentration of cells>

A sterilized cell concentration device was installed to construct a cell concentration system, and each of the solenoid valves, the connection tubes for designated positions, and a cassette unit in the pump were connected. The washing buffer bottle and the dilution buffer bottle each containing 500 mL of PBS buffer (Sigma-Aldrich, model name D8537) were connected to predetermined aseptic connection portions to start automated cell concentration operation. Comparative tests were performed in a similar manner using conventional circulation bottles (corresponding to the shape in Fig. 2) for comparison. The concentrations of the non-air gas held in the gas bag 6 were 5% CO₂, 20% O₂, and 78% N₂ and the air supply volume during filling is 0.5 L/min in air flow rate.

As a first step, the on-off valve 55 was kept open for 2.5 minutes, and the gas bag 6 was filled with the non-air gas having the aforementioned composition from the non-air gas supply source 54. Here, the volume of the non-air gas delivered is larger than the volume of the gas bag, but the excessive non-air gas is discharged to the air by the action of the check valve 56. Accordingly, the inner pressure of the gas bag 6 is kept at the atmospheric pressure.

Next, as a second step, 500 mL of the washing buffer was delivered from the wash bottle 42 to the flow-circulation chamber 2 and was passed through the cell concentration modules 12 to condition the module for the purpose of averaging the module pore sizes.

As a third step, the cell suspension was supplied from the sample bottle 46 to the flow-circulation chamber 2 in a volume of 500 mL and passed through the cell concentration module to concentrate the cell suspension. In order to maintain the trans-membrane pressure at 40 kPa, the flow rate was controlled by automatically controlling the voltage applied to the pump. When the volume of the liquid in the flow-circulation chamber 2 reached about 5 mL, the concentration operation was stopped.

As a fourth step, 250 mL of dilution buffer was delivered from the dilution buffer bottle 44 to the flow-circulation chamber 2 to dilute the cell concentrate, and then concentration operation was performed again. Once again, 25 0mL of dilution buffer was delivered to the flow-circulation chamber 2 to further perform the concentration. When the volume of liquid in the flow-circulation chamber 2 reached about 5 mL, the concentration operation was stopped.

As a fifth step, when the cell concentrate were collected in the collection bottle 25, the volume of the cell concentrate was 10 mL. The fully automatic steps described above were stopped, and the collection bottle was removed aseptically from the concentration device.

### <Manual concentration step for cells>

As a control experiment, cells were concentrated by manually operating centrifugation. The cell suspension identical to the one used along with the cell concentration device was distributed into two 250-ml centrifuge tubes (Corning Inc., model No. 430776) and centrifuged in a centrifuge (Hitachi Koki Co., Ltd., CR21F, rotor No.RPR12-2) for 5 min at 1,000 rpm (150G), and then the supernatant was removed, leaving about 1 ml.

### <Measurement method steps for concentrated cells>

The concentrated cells were subjected to centrifugation again in a single 15-ml tube to obtain cell pellet, which was re-dispersed in the medium of 2 ml, stained with trypan blue (Nacalai Tesque, Inc., Product No. 35525-02) using a conventional method, and counted by hemocytometer.

### <Evaluation Results for concentrated cells>

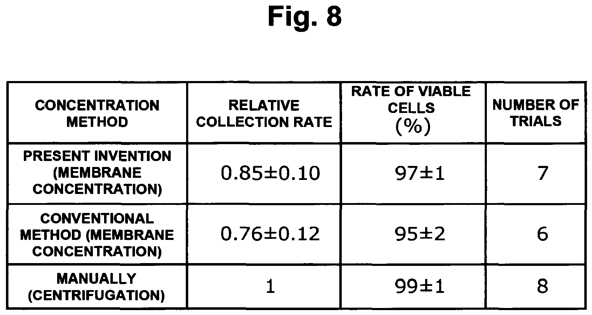

Fig. 8 summarizes the results of concentration for the suspension of cancer cells. A "relative collection rate" refers to a collection rate (= number of viable cells collected after concentration/number of viable cells supplied) obtained for each concentration method and normalized with the collection rate of the control experiments (centrifuge) as 1. The number of viable cells supplied is 1 by 10⁷ cells in all cases. The "Living cell ratio" was determined as the number of collected viable cells/the total number of collected cells by 100 (%). Fig. 8 shows the respective mean value and the standard deviation based on the "number of attempts". As a result, with respect to the conventional circulation bottles, when the circulation bottles of the present invention were used, the collection rate and the ratio o the viable cells were improved. An expected reason for this is that the bubbling in concentration of the cell suspension is prevented, so that the damage to the cells is reduced and the rate of collection of the viable cells is increased.

The control of the final volume is performed by weighing the concentrate, but the concentration was often excessive through the TFF membrane concentration by the conventional circulation bottles. This is because the control is performed using change in weight due to the falling droplets in the gas in the bottle during the measurement of the weight of the liquid, and the size of the droplets becomes uneven in the bubble-prone situation, and large errors in the fluid volume measurement occurs, which significantly deteriorates the adjustment of the final cell concentrate volume. The circulation bottles of the present invention achieves direct delivery into the liquid with a continuous flow, rather than falling in the gas, so that the detection of the liquid volume becomes a continuous flow and an error is less likely to occur, and exact cell concentrate volume can be achieved.

### [Industrial applicability]

According to the present invention, it is possible to provide cell concentration devices and cell concentration systems having a novel liquid flow-circulation chamber.

### [Denotation of symbols]

1 ... cell concentration device, 2 ... flow-circulation chamber, 3 ... lid, 4 ... atmospheric pressure regulating tube, 5 ... filter, 6 ... gas bag, 7 ... aspiration tube, 8 ... inlet tube, 9 ... opening, 10 ... pump, 11 ... liquid delivery tube, 12 ... cell concentration module, 13 ... separation membrane, 14 ... inlet port, 15 ... filtration port, 16 ... outlet port, 17 ... filtration tube, 18 ... valve, 19 ... discharge bag, 20 ... weight sensor, 21 ... pressure regulating valve, 22 ... controller, 23 ... direction of liquid delivery (upon concentration), 24 ... direction of liquid delivery (upon collection), 25 ... collection bottle, 26 ... on-off valve, 27 ... pressure sensor Pr, 28 ... pressure sensor Pp, 29 ... cell concentration device (prior art), 30 ... hydrophobic filter, 31 ... weight sensor, 32 ... cell concentration device, 33 ... on-off valve, 34 ... filter, 39 ... branch point, 40 ... cell concentration system, 41 ... pump, 42 ... wash bottle, 43 ... on-off valve, 44 ... dilution buffer bottle, 45 ... on-off valve, 46 ... sample bottle, 47 ... on-off valve, 48 ... on-off valve, 49 ... filter, 50 ... sterile connection part, 51, 52, 53 ... filter, 54 ... non-air gas supply source, 55 ... on-off valve, 56 ... check valve, 60 ... cell concentration system, 61 ... first cell concentration device, 62 ... on-off valve, 63 ... second cell concentration device, 64 ... on-off valve, 65 ... third cell concentration device, 70 ... cell concentration system, 71 ... first cell concentration module, 72 ... on-off valve, 73 ... on-off valve, 74 ... second cell concentration module, 75 ... on-off valve, 76 ... on-off valve, 77 ... third cell concentration module, 78 ... on-off valve

## Claims

1. A liquid flow-circulation chamber comprising a first tube and a second tube, the liquid flow-circulation chamber being hermetically closed,
the first tube and the second tube being connected with each other via a liquid delivery tube outside the liquid flow-circulation chamber,
the liquid delivery tube comprising a liquid delivery pump capable of feeding a liquid through the liquid delivery pump in both directions,
the first tube and the second tube each having an open end in a liquid in the liquid flow-circulation chamber, and
the first tube having an opening in a gas in the liquid flow-circulation chamber.

2. The liquid flow-circulation chamber according to Claim 1, further comprising a third tube connected to a gas bag.

3. The liquid flow-circulation chamber according to Claim 1 or 2, wherein an opening area of the opening is larger than a cross-sectional area of the first tube.

4. The liquid flow-circulation chamber according to any one of Claims 1 to 3, wherein the liquid contains cells.

5. A cell concentration device comprising a liquid flow-circulation chamber,
the liquid flow-circulation chamber having a first tube and a second tube, the first tube and the second tube being connected with each other via a liquid delivery tube outside the liquid flow-circulation chamber,
the liquid delivery tube comprising a liquid delivery pump and a cell concentration module, the liquid delivery pump being capable of feeding a liquid through the liquid delivery pump in both directions,
the first tube and the second tube each having an open end in a liquid in the liquid flow-circulation chamber,
the first tube having an opening in a gas in the liquid flow-circulation chamber, and
the liquid being a suspension of cells.

6. The cell concentration device according to Claim 5, further comprising a third tube connected to a gas bag.

7. The cell concentration device according to Claim 5 or 6, wherein the gas bag contains a gas containing 5 to 10% carbonate gas, and the suspension of the cells comprises a carbonate buffer.

8. The cell concentration device according to any one of Claims 5 to 7, wherein an opening area of the opening is larger than a cross-sectional area of first tube.

9. The cell concentration device according to any one of Claims 5 to 8, further comprising a discharge bag connected to the cell concentration module through an on-off valve or a check valve, the discharge bag being for holding a discharge from the cell concentration module.

10. A cell concentration system comprising a cell concentration device according to any one of Claims 5 to 9, in an attachable manner.

11. The cell concentration system according to Claim 10, further comprising a pressure sensor and/or a weight sensor.

12. The cell concentration system according to Claim 11, wherein
the pressure sensor is at least one pressure sensor selected from a group consisting of a pressure sensor for detecting a pressure in the upstream of the cell concentration module, a pressure sensor for detecting a pressure in the downstream of the cell concentration module, and a pressure sensor for detecting a pressure of a filtrate of the cell concentration module.

13. The cell concentration system according to any one of Claims 10 to 12, further comprising:
a sample container for supplying the cell suspension to the liquid flow-circulation chamber; and
a collection container for collecting the concentrated cell suspension.
